# EUROPÄISCHE PATENTSCHRIFT

(11) **EP 1 721 585 B1**
(45) Veröffentlichungstag und Bekanntmachung des Hinweises auf die Patenterteilung: **30.05.2012**
(21) Anmeldenummer: 06008942.2
(22) Anmeldetag: 28.04.2006
(51) Int. Cl.: A61F 2/38

(54) **Gekoppelte Knieendoprothese**
Hinged knee prosthesis
Prothèse du genou accouplée

(30) Priorität: 09.05.2005 DE 102005022576; 09.05.2005 DE 102005022584
(43) Veröffentlichungstag der Anmeldung: 15.11.2006
(73) Patentinhaber: Aesculap AG, 78532 Tuttlingen (DE); Reignier, Bernard, 72170 Maresché (FR)
(72) Erfinder: Roussel, Charly, 5200 Chaumont (FR); Miehlke, Rolf K., 48167 Münster (DE); Reignier, Bernard, 72170 Maresché (FR); Pfitscher, Klaus, 78194 Immendingen (DE)
(74) Vertreter: Hoeger, Stellrecht & Partner Patentanwälte

(56) Entgegenhaltungen:
- EP-A- 0 716 839
- EP-B- 0 400 045
- FR-A- 2 771 283
- US-A- 5 370 701
- US-A1- 2003 009 228

## Beschreibung

Die Erfindung betrifft eine gekoppelte Knieendoprothese mit einem eine obere Lagerfläche aufweisenden Tibiateil, einem auf der Lagerfläche aufliegenden, auf dieser um eine senkrecht auf der Lagerfläche stehende Drehachse verdrehbaren, auf seiner Oberseite Lagerschalen für ein Femurteil aufweisenden Meniskusteil, mit einem Femurteil mit sich in den Lagerschalen abstützenden Gleitflächen, mit einem das Femurteil mit einem Lagerzapfen gelenkig verbindenden Scharniergelenk und mit einer Lagerhülse im Tibiateil, die koaxial zu der Drehachse des Meniskusteils am Tibiateil angeordnet ist, das Meniskusteil durchsetzt und in die der Lagerzapfen frei drehbar und axial verschieblich eintaucht.

Bei diesem Typ von Knie-Totalendoprothesen handelt es sich um Prothesen, die durch das Scharniergelenk über einen Kopplungsmechanismus verbunden sind. Dieser hohe Kopplungsgrad erlaubt es, daß auch hohe Instabilitäten des Kniegelenks mit diesem Prothesentyp zuverlässig versorgt werden können.

Derartige Prothesen sind beispielsweise aus der EP 0400 045 B1 oder der US 5,370,701 bekannt.

Neben der Beugung ist es bei Knieendoprothesen dieser Bauart auch möglich, das Femurteil gegenüber dem Tibiateil um die Drehachse gegeneinander zu verdrehen, dabei verdreht sich das Meniskusteil gegenüber dem Tibiateil, und das Femurteil bleibt in den Lagerschalen des Meniskusteils abgestützt. Eine Begrenzung dieser Drehbewegung erfolgt bei Endoprothesen dieser Bauart entweder gar nicht oder dadurch, daß die Verdrehung des Meniskusteils gegenüber dem Tibiateil durch entsprechende Anschläge begrenzt wird (US 5,370,701). Dabei besteht aber die Gefahr, daß auch bei gegen einen Anschlag verdrehtem Tibiateil das Femurteil gegenüber dem Tibiateil noch weiter verdreht wird, allerdings verlassen dann die Gleitflächen des Femurteils die Lagerschalen des Meniskusteils und die flächige Abstützung der Gleitflächen in den Lagerschalen geht verloren. Dies kann zu erhöhter Abnützung führen, da nur noch Punkt- oder Linienkontakt zwischen den Gleitflächen und den Lagerschalen besteht.

Dieselbe Schwierigkeit ergibt sich bei anders gebauten Konstruktionen, bei denen eine Drehung von Femurteil und Tibiateil ausschließlich dadurch ermöglicht wird, daß bei unverdrehbarem Tibiateil die Gleitflächen des Femurteils bei dessen Verdrehung aus den Lagerschalen des Meniskusteils austreten.

Es sind weiterhin Knieendoprothesen dieses Typs bekannt, bei denen eine Drehsicherung zwischen Femurteil und einem drehfesten Meniskusteil dadurch erfolgt, daß das Femurteil in der Streckstellung der Endoprothese mit einem entsprechend geformten Vorsprung in eine Vertiefung des Meniskusteils eingreift. Eine solche Drehbegrenzung wirkt aber nur in der Streckstellung, sobald das Femurteil gegenüber dem Tibiateil gebeugt wird, wird diese Drehbegrenzung aufgehoben und die beiden Teile sind unbegrenzt gegeneinander verdrehbar (AXEL II Prothese der Firma Aesculap France S.A.; RT-PLUS Solution Prothese der Firma Plus Endoprothetic; Endo-Modell Rotationskniegelenk der Firma Waldemar Link).

Es ist Aufgabe der Erfindung, bei einer gekoppelten Knieendoprothese der gattungsgemäßen Art eine Drehbegrenzung zwischen Femurteil und Tibiateil zu schaffen, die sicherstellt, daß der Kontakt zwischen den Gleitflächen und den Lagerschalen von Femurteil beziehungsweise Meniskusteil erhalten bleibt und die über einen größeren Beugebereich von Femurteil und Tibiateil wirksam ist.

Diese Aufgabe wird bei einer gekoppelten Knieendoprothese der eingangs beschriebenen Art erfindungsgemäß dadurch gelöst, daß das Meniskusteil um die Drehachse anschlagfrei drehbar ist, daß die Lagerhülse um die Drehachse unverdrehbar an dem Tibiateil gehalten ist und daß die Lagerhülse an ihrem dem Tibiateil abgewandten oberen Ende seitliche Anschlagflächen aufweist, an denen das Femurteil oder an ihm gehaltene Teile bei einer Drehung des Femurteils um die Drehachse anschlagen und damit den Drehwinkel des Femurteils gegenüber dem Tibiateil bei einer Drehung um die Drehachse begrenzen.

Bei einer solchen Ausgestaltung bleibt der Kontakt zwischen Gleitflächen des Femurteils und Lagerschalen des Meniskusteils aufgrund der freien Drehbarkeit des Tibiateils in jedem Fall erhalten, andererseits wird die Verdrehung des Femurteils gegenüber dem Tibiateil durch die am Tibiateil drehfest gehaltene Lagerhülse begrenzt, die mit ihren Anschlagflächen mit dem Femurteil selbst oder mit an ihm gehaltenen Teilen in Kontakt kommt, wenn der maximale Drehwinkel erreicht ist. Ein solcher Anschlag kann über einen größeren Beugebereich von Femurteil und Tibiateil erfolgen und stellt somit über einen großen Beugebereich sicher, daß maximale Drehwinkel nicht überschritten werden.

Die Anschlagflächen der Lagerhülse können eben ausgebildet sein.

Weiterhin kann vorgesehen sein, daß diese Anschlagflächen parallel zur Drehachse verlaufen.

Besonders vorteilhaft ist es, wenn der Abstand der Anschlagflächen von der Mitte der Lagerhülse aus zu ihrer Außenseite hin abnimmt.

Insbesondere können die Anschlagflächen dabei gegenüber eine senkrechten Mittelebene der Lagerhülse um einen Winkel geneigt sein, der dem maximalen Drehwinkel des Femurteils gegenüber dem Tibiateil entspricht. Dies führt dazu, daß entsprechende Anlageflächen am Femurteil oder an einem am Femurteil gehaltenen Teil bei Erreichen des maximalen Drehwinkels flächig an diese Anschlagflächen anschlagen, dadurch wird der Verschleiß minimiert.

Bei einer bevorzugten Ausführungsform ist vorgesehen, daß die Lagerhülse in eine Aufnahmebuchse des Tibiateils um deren Längsachse frei drehbar eingesteckt ist und daß beim Einstecken Vor- und Rücksprünge an der Lagerhülse und am Tibiateil ineinandergreifen und die Lagerhülse dadurch in der Aufnahmebuchse gegen eine Drehung um die Längsachse sichern. Die drehfeste Verbindung zwischen Lagerhülse und Tibiateil wird also einfach durch die Vor- und Rücksprünge erreicht, die beim Einschieben der Lagerhülse in die Aufnahmebuchse formschlüssig ineinandergreifen.

Es ist günstig, wenn die Aufnahmebuchse in einem Ringflansch auf der oberen Lagerfläche des Tibiateils endet, der als Drehlager für das Meniskusteil dient.

Dieser Ringflansch kann nach oben abstehende Vorsprünge tragen, die zur Drehsicherung in entsprechend geformte Rücksprünge der Lagerhülse eingreifen.

Besonders günstig ist es, wenn die Lagerhülse zu einer senkrechten Ebene symmetrisch ausgebildet ist und wahlweise in einer von zwei um 180° verdrehten Stellungen drehfest in die Aufnahmebuchse einsetzbar ist.

Bei einer ersten bevorzugten Ausführungsform ist vorgesehen, daß das Femurteil selbst Anlageflächen trägt, die durch Anlage an den Anschlagflächen der Lagerhülse die Drehung des Femurteils relativ zum Tibiateil begrenzen.

Die Anlagefläche des Femurteils verläuft dabei vorzugsweise koaxial zur Schwenkachse des Scharniergelenks.

Bei einer anderen bevorzugten Ausführungsform ist dagegen vorgesehen, daß zwischen das Femurteil und den Lagerzapfen auf beiden Seiten desselben je ein eine Lagerwelle des Scharniergelenks umgebende Lagerscheibe eingelegt ist, die in einem über die Lagerwelle vorstehenden, diese konzentrisch umgebenden Bereich Anlageflächen trägt, die durch Anlage an den Anschlagflächen der Lagerhülse die Drehung des Femurteils relativ zum Tibiateil begrenzen.

Die Lagerscheiben werden vorzugsweise um die Schwenkachse unverdrehbar am Femurteil gehalten.

Eine besonders günstige Ausgestaltung ergibt sich, wenn der Abstand der Anlageflächen von den Anschlagflächen bei gegenüber dem Tibiateil unverdrehtem Femurteil bei verschiedenen Schwenkwinkel des Femurteils gegenüber dem Lagerzapfen und damit gegenüber der Lagerhülse unterschiedlich ist. Diese Ausgestaltung ermöglicht es, durch unterschiedliche Wahl dieses Abstandes den maximalen Drehwinkel festzulegen, der bei einem bestimmten Beugewinkel zwischen Femurteil und Tibiateil zugelassen wird.

So ist es vorteilhaft, wenn der Abstand zwischen den Anlageflächen und den Anschlagflächen von einer Streckstellung des Femurteils relativ zum Tibiateil zu einer Beugestellung hin zunimmt. Damit wird bei zunehmender Beugung auch der maximale Drehwinkel zwischen Femurteil und Tibiateil größer, dies entspricht auch den anatomischen Verhältnissen.

Insbesondere kann vorgesehen sein, daß die Anlageflächen bei vollständiger Streckung des Femurteils gegenüber der Lagerhülse dicht an den Anschlagflächen des Tibiateiles anliegen. Mit anderen Worten wird bei vollständig gestrecktem Kniegelenk eine Drehung um die Drehachse vollständig verhindert, sobald das Femurteil gegenüber dem Tibiateil gebeugt wird, wird jedoch eine Drehbewegung zugelassen, deren Größe vom Abstand der Anlageflächen und der Anschlagflächen in der jeweiligen Beugestellung abhängt.

Eine besonders vorteilhafte Ausgestaltung ergibt sich, wenn die Anlagefläche als koaxial zur Lagerwelle des Scharniergelenkes verlaufende stetig ansteigende Rampe ausgebildet ist. Dadurch ergibt sich ausgehend von der Streckstellung über den Beugewinkel eine stetige Zunahme des maximalen Drehwinkels, wobei durch die Ausgestaltung der Steigung der Rampe dem Konstrukteur eine volle Gestaltungsmöglichkeit über die Abhängigkeit zwischen maximalem Drehwinkel und Beugewinkel zur Verfügung steht.

Es ist günstig, wenn die Lagerscheiben aus Kunststoff bestehen, insbesondere aus Polyethylen.

Die Lagerscheiben können die Lagerwelle des Scharniergelenks mit einem Ringflansch umgeben, der in eine Lageröffnung des Femurteils eingreift. Damit bilden die Lagerscheiben zusätzlich auch eine Lagerung für die Lagerwelle im Femurteil aus.

Bei einer besonders bevorzugten Ausführungsform der eingangs beschriebenen Knieendoprothese ist vorgesehen, daß zur Anpassung der Bauhöhe der Knieendoprothese an die anatomischen Gegebenheiten Meniskusteile unterschiedlicher Höhe und für jedes Meniskusteil eine Lagerhülse mit an die Höhe des jeweiligen Meniskusteils angepaßter Länge vorgesehen sind.

Durch die Auswahl eines Meniskusteils der geeigneten Höhe kann der Abstand zwischen Femurteil und Tibiateil eingestellt werden. Zu jedem Meniskusteil mit einer bestimmten Bauhöhe gehört eine bestimmte Lagerhülse mit einer Länge, die an die Höhe des jeweiligen Meniskusteils angepaßt ist, so daß also bei einem dickeren Meniskusteil auch eine längere Lagerhülse verwendet wird. Dadurch wird sichergestellt, daß der Lagerzapfen des Scharniergelenks auch bei unterschiedlicher Höhe des Meniskusteils in gleicher Weise geführt wird, es geht also nicht durch die Zunahme der Höhe des Meniskusteils ein Teil der Führungslänge verloren.

Insbesondere kann vorgesehen sein, daß die Lagerhülsen unterschiedlicher Länge Anschläge aufweisen, durch die sich eine gleiche Eintauchtiefe der Lagerhülsen in eine Aufnahmebuchse des Tibiateils ergibt. Die Lagerhülse wird also unabhängig von der jeweiligen Länge der Lagerhülse über die gleiche Eintauchtiefe in der Aufnahmebuchse des Tibiateils gehalten, und die Lagerhülse selbst bietet dem Lagerzapfen des Scharniergelenks unabhängig von ihrer eigenen Länge in jedem Fall eine gleich große Eintauchtiefe und damit die gleiche Führung.

Insbesondere kann die Länge der Lagerhülsen so gewählt sein, daß diese bei Meniskusteilen unterschiedlicher Höhe nach oben gleich weit über die Meniskusteile hervorstehen.

Es ist günstig, wenn die Lagerhülsen drehfest an den Tibiateilen gehalten sind, die drehfesten Lagerhülsen können dann gleichzeitig durch geeignete Anschlagflächen als Drehsicherung für das Femurteil dienen.

Bei einer bevorzugten Ausführungsform ist vorgesehen, daß die Lagerhülse einen zylindrischen unteren Abschnitt und einen oberen Kopf aufweist und daß bei Lagerhülsen unterschiedlicher Länge die Länge des zylindrischen Abschnitts gleich ist und die Längenänderung allein durch eine Längenänderung des Kopfes erreicht ist.

Die nachfolgende Beschreibung bevorzugter Ausführungsformen der Erfindung dient im Zusammenhang mit der Zeichnung der näheren Erläuterung. Es zeigen:

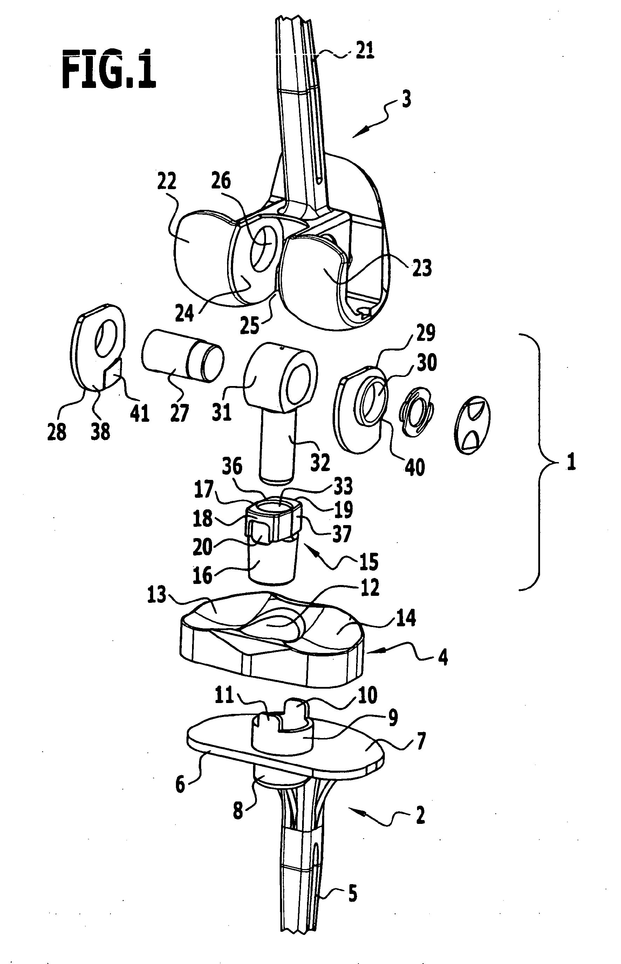

- Figur 1:: eine Explosionsdarstellung einer gekoppelten Knieendoprothese;
- Figur 2:: eine perspektivische Ansicht eines Teils des Scharniergelenks der Endoprothese der Figur 1 in gestreckter Stellung;
- Figur 3:: eine Schnittansicht längs Linie 3-3 in Figur 2;
- Figur 4:: eine Ansicht ähnlich Figur 2 bei um etwa 80° gebeugtem Kniegelenk;
- Figur 5:: eine Schnittansicht längs Linie 5-5 in Figur 4;
- Figur 6:: eine perspektivische Explosionsansicht der unteren Teile der Knieendoprothese der Figur 1 mit einem niedrigen Meniskusteil und einer kurzen Lagerhülse und
- Figur 7:: eine Ansicht ähnlich Figur 6 mit einem hohen Meniskusteil und einer entsprechend längeren Lagerhülse.

Die in der Zeichnung dargestellte Knieendoprothese 1 umfaßt ein Tibiateil 2, ein Femurteil 3 und ein zwischen den beiden angeordnetes Meniskusteil 4.

Am Tibiateil 2 ist an der Oberseite eines Haltezapfens 5, der in den Markraum eines Tibiaknochens eingeschoben wird, eine quer zur Längsrichtung des Haltezapfens 5 verlaufende Tibiaplatte 6 gehalten, deren ebene Oberseite eine Lagerfläche 7 für das an seiner Unterseite ebene Meniskusteil 4 bildet. Die Tibiaplatte 6 wird von einer zylindrischen Aufnahmebuchse 8 durchsetzt, deren Längsachse senkrecht zur Lagerfläche 7 verläuft und die die Lagerfläche 7 in Form eines Ringflansches 9 überragt. Auf zwei gegenüberliegenden Seiten trägt dieser Ringflansch 9 zwei nach oben abstehende Vorsprünge 10, 11.

Das Meniskusteil 4, das im Gegensatz zu dem metallischen Tibiateil 2 und dem metallischen Femurteil 3 aus einem Kunststoff besteht, insbesondere aus Polyethylen, überdeckt im wesentlichen die gesamte Lagerfläche 7 und weist eine zentrale, im Querschnitt kreisförmige Durchbrechung 12 auf, die mit ihrer Innenwand an der Außenseite des Ringflansches 9 dicht anliegt, so daß der Ringflansch 9 ein Drehgelenk für das Meniskusteil 4 ausbildet, dieses ist somit auf der Lagerfläche 7 um eine Drehachse verdrehbar, die mit der Längsachse der Aufnahmebuchse 8 zusammenfällt.

Zu beiden Seiten der Durchbrechung 12 sind auf der Oberseite des Meniskusteils 4 jeweils eine konkave Lagerschale 12, 13 eingearbeitet.

In die Aufnahmebuchse 8 ist von oben her eine Lagerhülse 15 eingesteckt, die einen zylindrischen unteren Abschnitt 16 und einen annähernd quaderförmigen Kopf 17 aufweist. An zwei gegenüberliegenden Seiten 18, 19 des Kopfes sind Ausnehmungen 20 eingearbeitet, die in ihrer Form komplementär zu den Vorsprüngen 10, 11 ausgebildet und nach unten offen sind. Beim Einstecken der Lagerhülse 15 in die Aufnahmebuchse 8 treten die Vorsprünge 10, 11 in die entsprechenden Ausnehmungen 20 des Kopfes 17 ein und legen dadurch die Lagerhülse 15 drehfest in der Aufnahmebuchse 8 fest. Der Kopf 17 ragt dabei über das Meniskusteil 4 nach oben hervor.

Das Femurteil 3 weist in ähnlicher Weise wie das Tibiateil 2 einen Haltezapfen 21 auf, der in den Markraum des Femurknochens eingeschoben wird, und der an einem Ende zwei im Abstand zueinander und parallel zueinander verlaufende gebogene Gleitflächen 22, 23 trägt, die bei montierter Knieendoprothese in die Lagerschalen 13, 14 eintauchen und sich in diesen abstützen.

Zwischen den beiden Gleitflächen 22, 23 ist eine durch zwei senkrechte Seitenwände 24 begrenzte Lagerkammer 25 ausgebildet. Die beiden Seitenwände 24 weisen je eine Lageröffnung 26 auf, die der Lagerung einer die Lagerkammer 25 quer durchsetzenden Lagerwelle 27 dienen. Zur Lagerung der Lagerwelle 27 werden zwei Lagerscheiben 28, 29 auf die Lagerwelle 27 aufgesteckt, diese umgeben die Lagerwelle 27 mit einem seitlich von den Lagerscheiben 28, 29 abstehenden ringflanschförmigen Bund 30, der in die Lageröffnungen 26 hineinragt und somit die Lagerwelle definiert in den Lageröffnungen 26 hält. Diese Lagerscheiben sind vorzugsweise aus einem Kunststoff gefertigt, beispielsweise aus Polyethylen.

Zwischen den beiden Lagerscheiben 28, 29 ist auf der Lagerwelle 27 mittels eines die Lagerwelle 27 umgebenden Lagerringes 31 ein radial von diesem abstehender Lagerzapfen 32 gelagert, der in den komplementär zu ihm ausgebildeten Innenraum 33 der Lagerhülse 15 eintaucht. Auf diese Weise werden das Femurteil 3 einerseits und das Tibiateil 2 andererseits über ein Scharniergelenk miteinander schwenkbar verbunden. Dieses Scharniergelenk ist jedoch in seiner Höhe relativ zum Tibiateil 2 verschiebbar, da der Lagerzapfen 32 in der Lagerhülse 15 in axialer Richtung frei verschiebbar ist. Der Abstand von Femurteil 3 und Tibiateil 2 wird durch die Anlage der Gleitflächen 22 und 23 an den Lagerschalen 13 beziehungsweise 14 bestimmt.

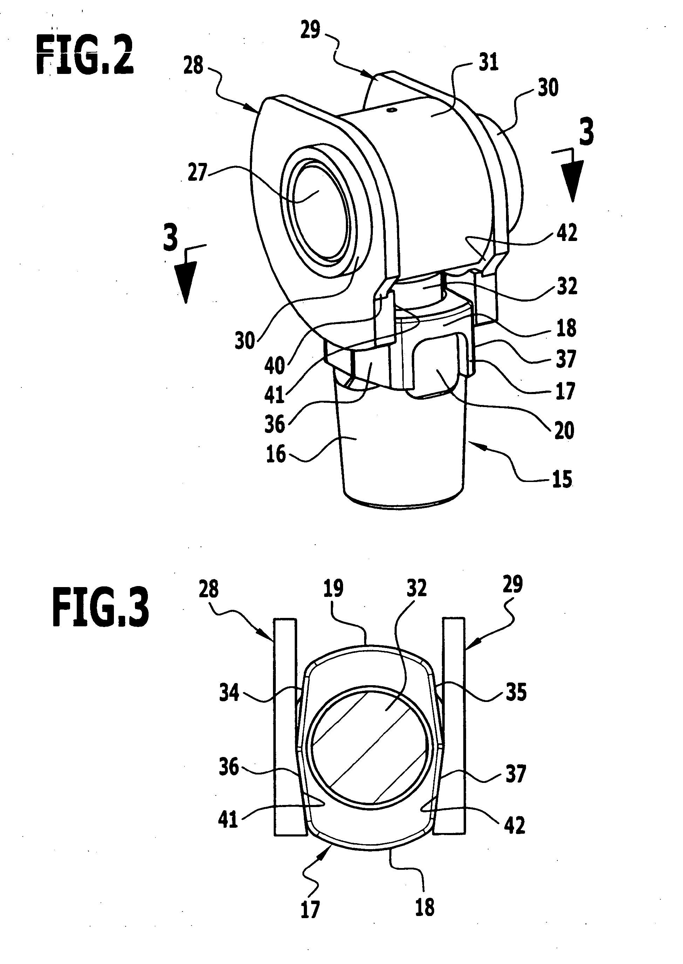

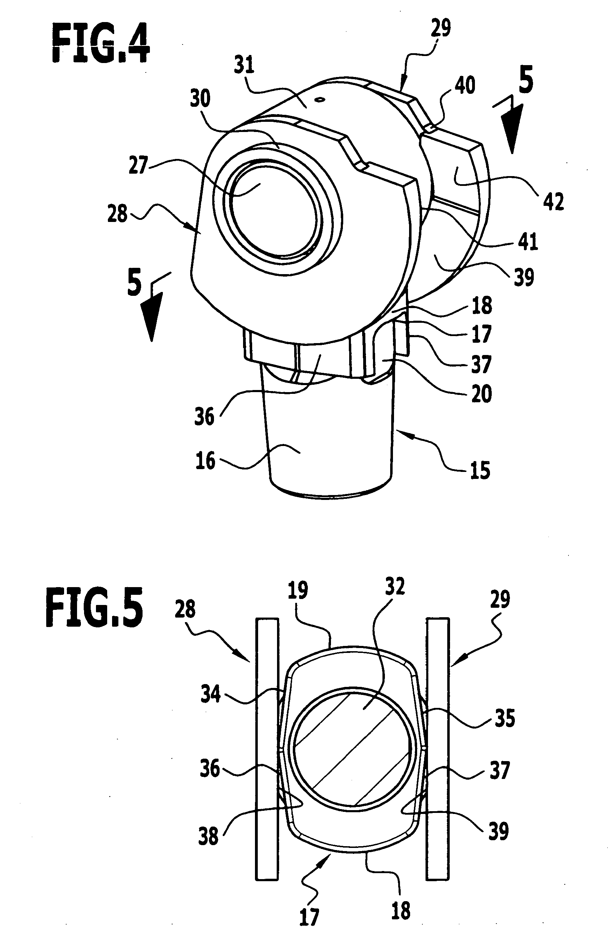

Der Kopf 17 der Lagerhülse 15 ragt in die Lagerkammer 25 hinein und steht mit seinen einander gegenüberliegenden Seitenflächen 34, 35 den Lagerscheiben 28, 29 gegenüber (Figuren 2 bis 4). Die Seitenflächen 34 und 35 des Kopfes 17 sind dabei in gleicher Weise in zwei ebene Teilflächen unterteilt, die jeweils von der Mitte des Kopfes 17 aus schräg zu den Seiten 18 und 19 des Kopfes 17 verlaufen, jeweils zwei der einander gegenüberliegenden Teilflächen 36, 37 bilden Anschlagflächen aus, die bei einer Verdrehung des Femurteils 3 um die durch die Lagerhülse 15 definierte Drehachse relativ zum Tibiateil 2 an einer der Lagerscheiben 28 beziehungsweise 29 anschlagen und damit die Drehbewegung begrenzen.

Wenn Lagerscheiben 28, 29 verwendet werden, die längs ihres Umfanges dieselbe Dicke haben, wird dieser maximale Drehwinkel für alle Beugewinkel des Femurteils 3 gegenüber dem Tibiateil 2 gleich sein, also von der vollständigen Streckung bis zur vollständigen Beugung. Die dem Kopf 17 zugewandte Innenseite der Lagerscheiben 28, 29 bildet dann jeweils eine Anlagefläche 38, 39 aus, die an einer der Teilflächen 36 oder 37 des Kopfes 17 anschlägt.

Bei der Ausgestaltung der Figuren 2 bis 4 ist eine spezielle Formgebung der Lagerscheiben 28, 29 vorgesehen. Diese Lagerscheiben 28, 29 sind zunächst gegen eine Drehung um die durch die Lagerwelle 27 definierte Schwenkachse gesichert am Femurteil 3 gehalten, dies kann erfolgen durch einen in der Zeichnung nicht dargestellten Vorsprung am Femurteil 3, der in einen entsprechenden Rücksprung 40 am Umfang der Lagerscheiben 28, 29 eingreift. Die Anlageflächen 38 und 39 der Lagerscheiben 28 und 29 verlaufen koaxial zur Schwenkachse des Scharniergelenks und angrenzend an den Umfang der Lagerwelle 27, und diese Anlageflächen 38, 39 sind bei den Ausführungsbeispielen der Figuren 2 bis 5 nach Art einer stetig ansteigenden Rampe 41, 42 ausgebildet, die zu dem Rücksprung 40 hin kontinuierlich ansteigt. Die Richtung des Anstieges und die Höhe der Rampen 41 und 42 sind dabei derart gewählt, daß bei vollständig gestrecktem Knie, also in der Streckstellung, die Rampen 41 und 42 an den Teilflächen 36, 37 des Kopfes 17 anliegen (Figuren 2 und 3), so daß bei vollständiger Streckung eine Drehung des Femurteils 3 gegenüber dem Tibiateil 2 vollständig unterbunden wird. Die Steigung der Rampe 41 ist dabei vorzugsweise so gewählt, daß sie mit der Neigung der Teilflächen 36, 37 übereinstimmt, so daß eine flächige Anlage erzielt wird.

Beim Verschwenken des Femurteils 3 gegenüber dem Tibiateil 2 und damit beim Verschwenken der Lagerscheiben 28, 29 gegenüber dem Kopf 17 vergrößert sich der Abstand zwischen der Oberseite der Rampen 41, 42 und den Teilflächen 36, 37, so daß eine begrenzte Drehung des Femurteils 3 gegenüber dem Tibiateil 2 um die Drehachse möglich wird, die Größe des zugelassenen Drehwinkels hängt dabei von der Formgebung der Rampe 41, 42 ab, die nicht unbedingt in Umfangsrichtung eben ausgebildet sein muß, sondern diese kann entsprechend den anatomischen Gegebenheiten auch eine von einer Ebene abweichenden Verlauf aufweisen. Der Konstrukteur hat durch die Formgebung der Rampe die Möglichkeit, die Größe des maximalen Drehwinkels in Abhängigkeit vom Beugewinkel zu bestimmen.

Die Rampen 41 und 42 gehen an dem dem Rücksprung 40 gegenüberliegenden Ende in die normale Innenfläche der Lagerscheiben 28, 29 über, so daß bei vollständiger Beugung der maximale Drehwinkel freigegeben wird.

Entsprechend den anatomischen Gegebenheiten kann mit der beschriebenen Konstruktion die Drehung des Femurteils gegenüber dem Tibiateil in der Strekkung vollständig unterbunden werden und dann bei zunehmender Streckung allmählich bis zu einem maximalen Drehwinkel freigegeben werden. Dabei ist immer sichergestellt, daß die Gleitflächen 22, 23 des Femurteils 3 vollständig in den Lagerschalen 13, 14 des Meniskusteils 4 verbleiben, da dieses frei drehbar auf dem Tibiateil 2 gelagert ist und sich der jeweiligen Drehstellung des Femurteils 3 ohne weiteres anpaßt.

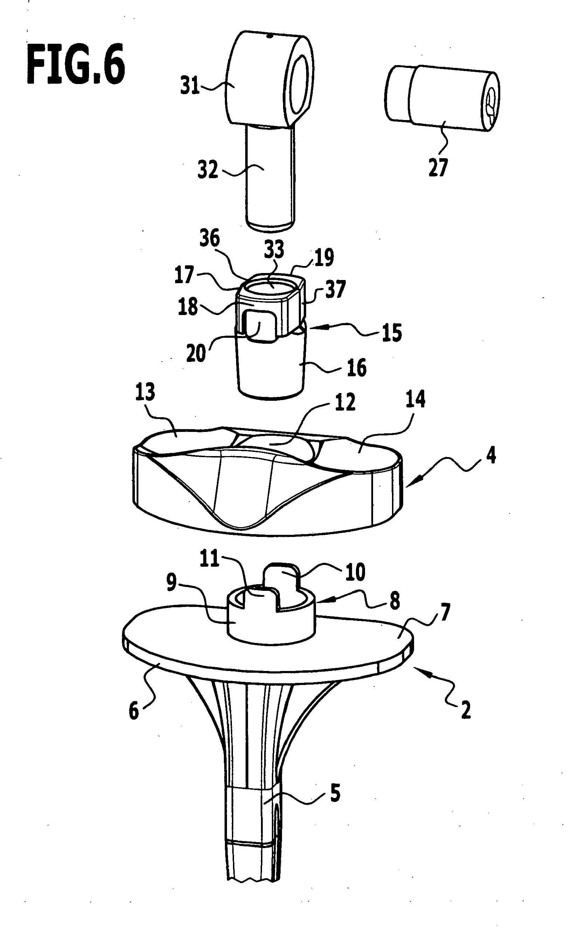

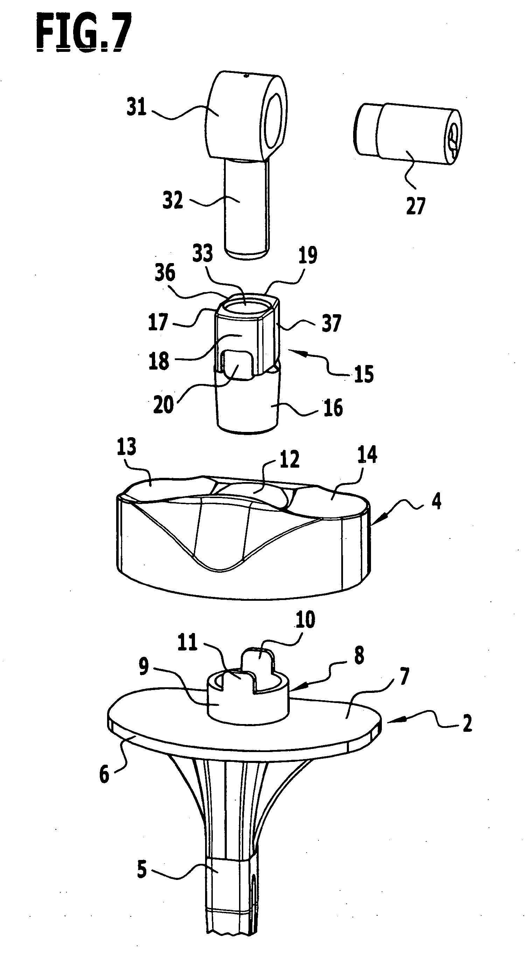

Wie aus den Darstellungen der Figuren 6 und 7 deutlich wird, können die Meniskusteile 4 in unterschiedlicher Höhe Verwendung finden. Im dargestellten Ausführungsbeispiel sind in den Figuren 6 und 7 Meniskusteile 4 mit zwei unterschiedlichen Höhen dargestellt, es versteht sich aber, daß eine größere Anzahl von Meniskusteilen 4 mit jeweils verschiedener Bauhöhe Verwendung finden können, so daß dem Operateur ein Bausatz zur Verfügung steht, aus dem er den Meniskusteil 4 der gewünschten Höhe auswählen kann.

Jedem Meniskusteil 4 einer bestimmten Bauhöhe ist eine Lagerhülse 15 einer bestimmten Länge zugeordnet, dabei unterscheiden sich die Lagerhülsen 15 nicht in der Länge des zylindrischen unteren Abschnitts 16, sondern nur in der Baulänge des Kopfes 17. Dieser ist entsprechend der Zunahme der Höhe des Meniskusteils 4 verlängert, so daß die Lagerhülse 15 in allen Fällen gleich weit über die Oberseite des Meniskusteils 4 und damit deren Lagerschalen 13, 14 hervorsteht. Dadurch wird erreicht, daß der Lagerzapfen 32 des Lagerrings 31 unabhängig von der Bauhöhe der Meniskusteils 4 immer gleich weit in die Lagerhülse 15 eintaucht und in dieser in gleicher Weise geführt wird.

Der Bausatz umfaßt also neben den unterschiedlich hohen Meniskusteilen 4 auch eine entsprechende Anzahl von Lagerhülsen 15 unterschiedlicher Länge, wobei deren Länge an die unterschiedliche Höhe der Meniskusteile 4 angepaßt ist.

## Patentansprüche

1. Gekoppelte Knieendoprothese mit einem eine obere Lagerfläche aufweisenden Tibiateil, einem auf der Lagerfläche aufliegenden, auf dieser um eine senkrecht auf der Lagerfläche stehende Drehachse verdrehbaren, auf seiner Oberseite Lagerschalen für ein Femurteil aufweisenden Meniskusteil, mit einem Femurteil mit sich in den Lagerschalen abstützenden Gleitflächen, mit einem das Femurteils mit einem Lagerzapfen gelenkig verbindenden Scharniergelenk und mit einer Lagerhülse im Tibiateil, die koaxial zu der Drehachse des Meniskusteils am Tibiateil angeordnet ist, das Meniskusteil durchsetzt und in die der Lagerzapfen frei drehbar und axial verschieblich eintaucht, **dadurch gekennzeichnet, daß** das Meniskusteil (4) relativ zum Tibiateil (2) um die Drehachse anschlagfrei drehbar ist, daß die Lagerhülse (15) um die Drehachse unverdrehbar an dem Tibiateil (2) gehalten ist und daß die Lagerhülse (15) an ihrem dem Tibiateil (2) abgewandten oberen Ende seitliche Anschlagflächen (36, 37) aufweist, an denen das Femurteil (3) oder an ihm gehaltene Teile (28, 29) bei einer Drehung des Femurteils (3) um die Drehachse anschlagen und damit den Drehwinkel des Femurteils (3) gegenüber dem Tibiateil (2) bei einer Drehung um die Drehachse begrenzen.

2. Prothese nach Anspruch 1, **dadurch gekennzeichnet, daß** die Anschlagflächen (36, 37) der Lagerhülse (15) eben sind.

3. Prothese nach Anspruch 1 oder 2, **dadurch gekennzeichnet, daß** die Anschlagflächen (36, 37) parallel zu der Drehachse verlaufen.

4. Prothese nach einem der voranstehenden Ansprüche, **dadurch gekennzeichnet, daß** der Abstand der Anschlagflächen (36, 37) von der Mitte der Lagerhülse (15) aus zu ihrer Außenseite (18, 19) hin abnimmt.

5. Prothese nach Anspruch 4, **dadurch gekennzeichnet, daß** die Anschlagflächen (36, 37) gegenüber einer senkrechten Mittelebene der Lagerhülse (15) um einen Winkel geneigt sind, der dem maximalen Drehwinkel des Femurteils (3) gegenüber dem Tibiateil (2) entspricht.

6. Prothese nach einem der voranstehenden Ansprüche, **dadurch gekennzeichnet, daß** die Lagerhülse (15) in eine Aufnahmebuchse (8) des Tibiateiles (2) um deren Längsachse frei drehbar eingesteckt ist und daß beim Einstecken Vor- und Rücksprünge (10, 11; 20) an der Lagerhülse (15) und am Tibiateil (2) ineinandergreifen und die Lagerhülse (15) **dadurch** in der Aufnahmebuchse (8) gegen eine Drehung um die Längsachse sichern.

7. Prothese nach Anspruch 6, **dadurch gekennzeichnet, daß** die Aufnahmebuchse (8) in einem Ringflansch (9) auf der oberen Lagerfläche (7) des Tibiateils (2) endet, der als Drehlager für das Meniskusteil (4) dient.

8. Prothese nach Anspruch 7, **dadurch gekennzeichnet, daß** der Ringflansch (9) nach oben abstehende Vorsprünge (10, 11) trägt, die zur Drehsicherung in entsprechend geformte Rücksprünge (20) der Lagerhülse (15) eingreifen.

9. Prothese nach einem der Ansprüche 6 bis 8, **dadurch gekennzeichnet, daß** die Lagerhülse (15) zu einer senkrechten Ebene symmetrisch ausgebildet ist und wahlweise in einer von zwei um 180° verdrehten Stellungen drehfest in die Aufnahmebuchse (8) einsetzbar ist.

10. Prothese nach einem der voranstehenden Ansprüche, **dadurch gekennzeichnet, daß** das Femurteil (3) selbst Anlageflächen trägt, die durch Anlage an den Anschlagflächen (36, 37) der Lagerhülse (15) die Drehung des Femurteils (3) relativ zum Tibiateil (2) begrenzen.

11. Prothese nach Anspruch 10, **dadurch gekennzeichnet, daß** die Anlagefläche des Femurteils (3) koaxial zur Schwenkachse des Scharniergelenks (27, 31) verläuft.

12. Prothese nach einem der Ansprüche 1 bis 9, **dadurch gekennzeichnet, daß** zwischen das Femurteil (3) und den Lagerzapfen (31, 32) auf beiden Seiten desselben je eine eine Lagerwelle (27) des Scharniergelenks umgebende Lagerscheibe (28, 29) eingelegt ist, die in einem über die Lagerwelle (27) vorstehenden, diese konzentrisch umgebenden Bereich Anlageflächen (38, 39; 41, 42) trägt, die durch Anlage an den Anschlagflächen (36, 37) der Lagerhülse (15) die Drehung des Femurteils (3) relativ zum Tibiateil (2) begrenzen.

13. Prothese nach Anspruch 12, **dadurch gekennzeichnet, daß** die Lagerscheiben (28, 29) um die Schwenkachse unverdrehbar am Femurteil (3) gehalten sind.

14. Prothese nach einem der Ansprüche 10 bis 13, **dadurch gekennzeichnet, daß** der Abstand der Anlageflächen (38, 39; 41, 42) von den Anschlagflächen (36, 37) bei gegenüber dem Tibiateil (2) unverdrehtem Femurteil (3) bei verschiedenen Schwenkwinkeln des Femurteils (3) gegenüber dem Lagerzapfen (31, 32) und damit gegenüber der Lagerhülse (15) unterschiedlich ist.

15. Prothese nach Anspruch 14, **dadurch gekennzeichnet, daß** der Abstand zwischen den Anlageflächen (41, 42) und den Anschlagflächen (36, 37) von einer Streckstellung des Femurteils (3) relativ zum Tibiateil (2) zu einer Beugestellung hin zunimmt.

16. Prothese nach Anspruch 15, **dadurch gekennzeichnet, daß** die Anlageflächen des Femurteils bei vollständiger Streckung des Femurteils gegenüber der Lagerhülse (15) dicht an den Anschlagflächen des Tibiateils (2) anliegen.

17. Prothese nach einem der Ansprüche 15 oder 16, **dadurch gekennzeichnet, daß** die Anlagefläche als koaxial zur Lagerwelle (27) des Scharniergelenks verlaufende stetig ansteigende Rampe (41, 42) ausgebildet ist.

18. Prothese nach einem der Ansprüche 12 bis 17, **dadurch gekennzeichnet, daß** die Lagerscheiben (28, 29) aus Kunststoff bestehen.

19. Prothese nach Anspruch 18, **dadurch gekennzeichnet, daß** die Lagerscheiben (28, 29) aus Polyethylen bestehen.

20. Prothese nach einem der Ansprüche 12 bis 19, **dadurch gekennzeichnet, daß** die Lagerscheiben (28, 29) die Lagerwelle (27) des Scharniergelenks mit einem Ringflansch (30) umgeben, der in eine Lageröffnung (26) des Femurteils (3) eingreift.

21. Gekoppelte Knieendoprothese, nach einem der voranstehenden Ansprüche, **dadurch gekennzeichnet, dass** zur Anpassung der Bauhöhe der Knieendoprothese (1) an die anatomischen Gegebenheiten Meniskusteile (4) unterschiedlicher Höhe und für jedes Meniskusteil (4) eine Lagerhülse (15) mit an die Höhe des jeweiligen Meniskusteils (4) angepasster Länge vorgesehen sind.

22. Prothese nach Anspruch 21, **dadurch gekennzeichnet, daß** die Lagerhülsen (15) unterschiedlicher Länge Anschläge (20) aufweisen, durch die sich eine gleiche Eintauchtiefe der Lagerhülsen (15) in eine Aufnahmebuchse (8) des Tibiateils (2) ergibt.

23. Prothese nach einem der Ansprüche 21 oder 22, **dadurch gekennzeichnet, daß** die Länge der Lagerhülsen (15) so gewählt ist, daß diese bei Meniskusteilen (4) unterschiedlicher Höhe nach oben gleich weit über die Meniskusteile (4) hervorstehen.

24. Prothese nach einem der Ansprüche 21 bis 23, **dadurch gekennzeichnet, daß** die Lagerhülsen (15) drehfest an den Tibiateilen (2) gehalten sind.

25. Prothese nach einem der Ansprüche 21 bis 24, **dadurch gekennzeichnet, daß** die Lagerhülsen (15) einen zylindrischen unteren Abschnitt (16) und einen oberen Kopf (17) aufweisen und daß bei Lagerhülsen (15) unterschiedlicher Länge die Länge des zylindrischen Abschnitts (16) gleich ist und die Längenänderung allein durch eine Längenänderung des Kopfes (17) erreicht ist.

## Claims

1. Coupled knee endoprosthesis with a tibia part having an upper bearing surface, a meniscus part, which lies on the bearing surface, is rotatable on this around a rotation axis, which is perpendicular to the bearing surface, and has bearing shells for a femur part on its upper surface, with a femur part with sliding surfaces supported in the bearing shells, with a hinge joint articulating the femur part to a journal pin and with a bearing sleeve in the tibia part, which is arranged on the tibia part coaxially to the rotation axis of the meniscus part, passes through the meniscus part and into which the journal pin extends in a freely rotatable and axially displaceable manner, **characterised in that** the meniscus part (4) is rotatable in relation to the tibia part (2) around the rotation axis without a stop means, that the bearing sleeve (15) is held on the tibia part (2) to be non-rotatable around the rotation axis and that at its upper end remote from the tibia part (2) the bearing sleeve (15) has lateral stop faces (36, 37), against which the femur part (3) or parts (28, 29) held thereon impinge during a rotation of the femur part (3) around the rotation axis and thus restrict the angle of rotation of the femur part (3) in relation to the tibia part (2) during a rotation around the rotation axis.

2. Prosthesis according to claim 1, **characterised in that** the stop faces (36, 37) of the bearing sleeve (15) are planar.

3. Prosthesis according to claim 1 or 2, **characterised in that** the stop faces (36, 37) extend parallel to the rotation axis.

4. Prosthesis according to one of the preceding claims, **characterised in that** the spacing of the stop faces (36, 37) decreases from the centre of the bearing sleeve (15) towards the outer surface (18, 19) thereof.

5. Prosthesis according to claim 4, **characterised in that** the stop faces (36, 37) are inclined in relation to a vertical central plane of the bearing sleeve (15) by an angle, which corresponds to the maximum angle of rotation of the femur part (3) in relation to the tibia part (2).

6. Prosthesis according to one of the preceding claims, **characterised in that** the bearing sleeve (15) is inserted into a receiving socket (8) of the tibia part (2) to be freely rotatable around the longitudinal axis thereof and that during insertion projections and recesses (10, 11; 20) on the bearing sleeve (15) and on the tibia part (2) intermesh and thus secure the bearing sleeve (15) in the receiving socket (8) against a rotation around the longitudinal axis.

7. Prosthesis according to claim 6, **characterised in that** the receiving socket (8) terminates in a ring flange (9) on the upper bearing surface (7) of the tibia part (2), which serves as a rotational bearing for the meniscus part (4).

8. Prosthesis according to claim 7, **characterised in that** the ring flange (9) bears upwardly protruding projections (10, 11), which engage into correspondingly shaped recesses (20) of the bearing sleeve (15) to secure against rotation.

9. Prosthesis according to one of claims 6 to 8, **characterised in that** the bearing sleeve (15) is configured symmetrically to a vertical plane and in one of two positions rotated by 180° can be selectively inserted into the receiving socket (8) to be fixed against rotation.

10. Prosthesis according to one of the preceding claims, **characterised in that** the femur part (3) itself bears abutment surfaces, which restrict the rotation of the femur part (3) in relation to the tibia part (2) by abutment against the stop faces (36, 37) of the bearing sleeve (15).

11. Prosthesis according to claim 10, **characterised in that** the abutment surface of the femur part (3) extends coaxially to the pivot axis of the hinge joint (27, 31).

12. Prosthesis according to one of claims 1 to 9, **characterised in that** inserted between the femur part (3) and the journal pin (31, 32) on both sides thereof is a respective bearing plate (28, 29), which surrounds a bearing shaft (27) of the hinge joint and in a region projecting over the bearing shaft (27) and concentrically surrounding this bears abutment surfaces (38, 39; 41, 42), which restrict the rotation of the femur part (3) in relation to the tibia part (2) by abutment against the stop faces (36, 37) of the bearing sleeve (15).

13. Prosthesis according to claim 12, **characterised in that** the bearing plates (28, 29) are held on the femur part (3) to be non-rotatable around the pivot axis.

14. Prosthesis according to one of claims 10 to 13, **characterised in that** the spacing of the abutment surfaces (38, 39; 41, 42) from the stop faces (36, 37) when the femur part (3) is not rotated in relation to the tibia part (2) is different with different swivel angles of the femur part (3) in relation to the journal pin (31, 32) and thus in relation to the bearing sleeve (15).

15. Prosthesis according to claim 14, **characterised in that** the spacing between the abutment surfaces (41, 42) and the stop faces (36, 37) from an extension position of the femur part (3) in relation to the tibia part (2) increases towards a flexion position.

16. Prosthesis according to claim 15, **characterised in that** with full extension of the femur part in relation to the bearing sleeve (15) the abutment surfaces of the femur part abut closely against the stop faces of the tibia part (2).

17. Prosthesis according to one of claims 15 or 16, **characterised in that** the abutment surface is configured as a constantly rising ramp (41, 42) extending coaxially to the bearing shaft (27) of the hinge joint.

18. Prosthesis according to one of claims 12 to 17, **characterised in that** the bearing plates (28, 29) are made of plastic.

19. Prosthesis according to claim 18, **characterised in that** the bearing plates (28, 29) are made of polyethylene.

20. Prosthesis according to one of claims 12 to 19, **characterised in that** the bearing plates (28, 29) surround the bearing shaft (27) of the hinge joint with a ring flange (30), which engages into a bearing opening (26) of the femur part (3).

21. Coupled knee endoprosthesis according to one of the preceding claims, **characterised in that** to match the structural height of the knee endoprosthesis (1) to the anatomical features meniscus parts (4) of different height are provided and a bearing sleeve (15) with a length matched to the height of the respective meniscus part (4) is provided for each meniscus part (4).

22. Prosthesis according to claim 21, **characterised in that** the bearing sleeves (15) of different length have stops (20), which result in an identical penetration depth of the bearing sleeves (15) into a receiving socket (8) of the tibia part (2).

23. Prosthesis according to one of claims 21 or 22, **characterised in that** the length of the bearing sleeves (15) is selected so that with meniscus parts (4) of different height said bearing sleeves project upwards by an equal amount above the meniscus parts (4).

24. Prosthesis according to one of claims 21 to 23, **characterised in that** the bearing sleeves (15) are held on the tibia parts (2) to be fixed against rotation.

25. Prosthesis according to one of claims 21 to 24, **characterised in that** the bearing sleeves (15) have a cylindrical lower section (16) and an upper head (17) and that with bearing sleeves (15) of different length the length of the cylindrical section (16) is the same and the change of length is achieved solely by a change of length of the head (17).

## Revendications

1. Endoprothèse de genou couplée, comprenant une partie tibiale, qui présente une surface de palier supérieure, une partie formant ménisque, qui s'appuie sur ladite surface de palier et est susceptible d'y tourner autour d'un axe de rotation normal à la surface de palier, et comportant sur son côté supérieur, des cavités de palier destinées à une partie de fémur, l'ensemble comprenant en outre une partie de fémur avec des surfaces de glissement, qui s'appuient dans les cavités de palier, et comprenant par ailleurs une articulation à charnière reliant de manière articulée la partie de fémur à un tourillon de palier, ainsi qu'un coussinet de palier dans la partie tibiale, qui est agencé sur la partie tibiale de manière coaxiale à l'axe de rotation de la partie formant ménisque, traverse la partie formant ménisque, et dans lequel s'engage le tourillon de palier de manière librement tournante et axialement coulissante,
**caractérisée en ce que** la partie formant ménisque (4) peut tourner par rapport à la partie tibiale (2) autour de l'axe de rotation, de manière libre de toute butée, **en ce que** le coussinet de palier (15) est maintenu non rotatif autour de l'axe de rotation sur la partie tibiale (2), et **en ce que** le coussinet de palier (15) présente, à son extrémité supérieure opposée à celle dirigée vers la partie tibiale (2), des surfaces de butée latérales (36, 37) contre lesquelles vient buter la partie de fémur (3) ou des pièces (28, 29) qui y sont supportées, lors d'une rotation de la partie de fémur (3) autour de l'axe de rotation, en limitant ainsi l'angle de rotation de la partie de fémur (3) par rapport à la partie tibiale (2) lors d'une rotation autour de l'axe de rotation.

2. Prothèse selon la revendication 1, **caractérisée en ce que** les surfaces de butée (36, 37) du coussinet de palier (15) sont planes.

3. Prothèse selon la revendication 1 ou la revendication 2, **caractérisée en ce que** les surfaces de butée (36, 37) s'étendent parallèlement à l'axe de rotation.

4. Prothèse selon l'une des revendications précédentes, **caractérisée en ce que** la distance d'espacement des surfaces de butée (36, 37) diminue à partir du centre du coussinet de palier (15) en direction de son côté extérieur (18, 19).

5. Prothèse selon la revendication 4, **caractérisée en ce que** les surfaces de butée (36, 37) sont inclinées par rapport à un plan médial vertical du coussinet de palier (15), d'un angle qui correspond à l'angle de rotation maximal de la partie de fémur (3) par rapport à la partie tibiale (2).

6. Prothèse selon l'une des revendications précédentes, **caractérisée en ce que** le coussinet de palier (15) est inséré dans une douille de réception (8) de la partie tibiale (2) de manière librement tournante autour de l'axe longitudinal de celle-ci, et **en ce que** lors de l'insertion, des protubérances et des cavités (10, 11 ; 20) sur le coussinet de palier (15) et sur la partie tibiale (2) s'engagent réciproquement les unes dans les autres et arrêtent ainsi le coussinet de palier (15) à l'encontre d'une rotation autour de l'axe longitudinal dans la douille de réception (8).

7. Prothèse selon la revendication 6, **caractérisée en ce que** la douille de réception (8) se termine, sur la surface de palier supérieure (7) de la partie tibiale (2), par une collerette annulaire (9) qui sert de palier de rotation pour la partie formant ménisque (4).

8. Prothèse selon la revendication 7, **caractérisée en ce que** la collerette annulaire (9) porte des protubérances (10, 11) faisant saillie vers le haut et qui s'engagent dans des cavités (20) de forme correspondante du coussinet de palier (15), en vue de réaliser l'arrêt de rotation.

9. Prothèse selon l'une des revendications 6 à 8, **caractérisée en ce que** le coussinet de palier (15) est de configuration symétrique par rapport à un plan vertical, et peut être inséré de manière fixe en rotation, au choix selon l'une de deux positions tournées de 180° l'une par rapport à l'autre, dans la douille de réception (8).

10. Prothèse selon l'une des revendications précédentes, **caractérisée en ce que** la partie de fémur (3) porte elle-même des surfaces d'appui, qui limitent la rotation de la partie de fémur (3) par rapport à la partie tibiale (2), en venant s'appuyer contre les surfaces de butée (36, 37) du coussinet de palier (15).

11. Prothèse selon la revendication 10, **caractérisée en ce que** la surface d'appui de la partie de fémur (3) s'étend de manière coaxiale à l'axe de pivotement de l'articulation à charnière (27, 31).

12. Prothèse selon l'une des revendications 1 à 9, **caractérisée en ce qu'**entre la partie de fémur (3) et le tourillon de palier (31, 32) est placé, des deux côtés de celui-ci, respectivement un disque de palier (28, 29) qui entoure un arbre de palier (27) de l'articulation à charnière, et porte, dans une zone dépassant de l'arbre de palier (27) et l'entourant de manière concentrique, des surfaces d'appui (38, 39 ; 41, 42) limitant la rotation de la partie de fémur (3) par rapport à la partie tibiale (2), en venant en appui contre les surfaces de butée (36, 37) du coussinet de palier (15).

13. Prothèse selon la revendication 12, **caractérisée en ce que** les disques de palier (28, 29) sont maintenus sur la partie de fémur (3) de manière non rotative autour de l'axe de pivotement.

14. Prothèse selon l'une des revendications 10 à 13, **caractérisée en ce que** la distance des surfaces d'appui (38, 39 ; 41, 42) aux surfaces de butée (36, 37), pour une partie de fémur (3) non tournée par rapport à la partie tibiale (2), est différente pour des angles de pivotement différents de la partie de fémur (3) par rapport au tourillon de palier (31, 32) et ainsi par rapport au coussinet de palier (15).

15. Prothèse selon la revendication 14, **caractérisée en ce que** la distance entre les surface d'appui (41, 42) et les surfaces de butée (36, 37) augmente à partir d'une position d'extension de la partie de fémur (3) par rapport à la partie tibiale (2), en allant vers une position de flexion.

16. Prothèse selon la revendication 15, **caractérisée en ce que** pour une extension totale de la partie de fémur par rapport au coussinet de palier (15), les surfaces d'appui de la partie de fémur s'appliquent de manière étroite contre les surfaces de butée de la partie tibiale (2).

17. Prothèse selon l'une des revendications 15 ou 16, **caractérisée en ce que** la surface d'appui est réalisée sous forme de rampe (41, 42) montante continue, s'étendant de manière coaxiale à l'arbre de palier (27) de l'articulation à charnière.

18. Prothèse selon l'une des revendications 12 à 17, **caractérisée en ce que** les disques de palier (28, 29) sont réalisés en matière plastique.

19. Prothèse selon la revendication 18, **caractérisée en ce que** les disques de palier (28, 29) sont réalisés en polyéthylène.

20. Prothèse selon l'une des revendications 12 à 19, **caractérisée en ce que** les disques de palier (28, 29) entourent l'arbre de palier (27) de l'articulation à charnière avec une collerette annuaire (30), qui s'engage dans une ouverture de palier (26) de la partie de fémur (3).

21. Endoprothèse de genou, couplée, selon l'une des revendications précédentes, **caractérisée en ce que** pour l'adaptation de la hauteur d'encombrement de l'endoprothèse de genou (1) aux conditions anatomiques, il est prévu des parties formant ménisque (4) de hauteur différente, et pour chaque partie formant ménisque (4) est prévu un coussinet de palier (15) d'une longueur adaptée à la hauteur de la partie formant ménisque (4) respectivement considérée.

22. Prothèse selon la revendication 21, **caractérisée en ce que** les coussinets de palier (15) de longueur différente, présentent des butées (20) desquelles résulte une profondeur de pénétration identique des coussinets de palier (15) dans une douille de réception (8) de la partie tibiale (2).

23. Prothèse selon l'une des revendications 21 ou 22, **caractérisée en ce que** la longueur des coussinets de palier (15) est choisie de façon à ce que ceux-ci, pour des parties formant ménisque (4) de hauteur différente, fassent saillie des parties formant ménisque (4) d'une valeur identique vers le haut.

24. Prothèse selon l'une des revendications 21 à 23, **caractérisée en ce que** les coussinets de palier (15) sont tenus de manière fixe en rotation sur les parties tibiales (2).

25. Prothèse selon l'une des revendications 21 à 24, **caractérisée en ce que** les coussinets de palier (15) présentent un tronçon inférieur cylindrique (16) et une tête supérieure (17), et **en ce que** pour des coussinets de palier (15) de longueur différente, la longueur du tronçon cylindrique (16) est identique, et la variation de longueur est obtenue uniquement par une variation de la longueur de la tête (17).
